# EUROPÄISCHE PATENTSCHRIFT

(11) **EP 2 512 596 B1**
(45) Veröffentlichungstag und Bekanntmachung des Hinweises auf die Patenterteilung: **17.08.2016**
(21) Anmeldenummer: 11702623.7
(22) Anmeldetag: 02.02.2011
(51) Int. Cl.: A61N 5/10, A61N 5/01

(54) **MEDIZINISCHES, MIT RÖNTGENSTRAHLEN ARBEITENDES GERÄT SOWIE VERFAHREN ZUM BETREIBEN EINES SOLCHEN**
MEDICAL DEVICE OPERATING WITH X-RAYS AND METHOD FOR OPERATING SAME
APPAREIL MÉDICAL À RAYONS X ET PROCÉDÉ DE FONCTIONNEMENT

(30) Priorität: 24.02.2010 DE 102010009019
(43) Veröffentlichungstag der Anmeldung: 24.10.2012
(73) Patentinhaber: Siemens Aktiengesellschaft, 80333 München (DE)
(72) Erfinder: HEID, Oliver, 91052 Erlangen (DE)
(86) Internationale Anmeldenummer: PCT/EP2011/051459
(87) Internationale Veröffentlichungsnummer: WO 2011/104075

(56) Entgegenhaltungen:
- EP-A1- 1 419 799
- WO-A1-00/74779
- WO-A1-2005/053794
- US-A1- 2005 089 141
- US-A1- 2006 153 330
- US-A1- 2009 080 603

## Beschreibung

Medizinisches, mit Röntgenstrahlen arbeitendes Gerät sowie Verfahren zum Betreiben eines medizinischen Diagnose-Geräts

Die Erfindung betrifft ein medizinisches, mit Röntgenstrahlen arbeitendes Gerät, insbesondere ein Strahlentherapiegerät, sowie ein Verfahren zum Betreiben eines medizinischen Diagnose-Geräts.

Entsprechende Geräte sind beispielsweise in der US 2006/153330 A1, EP 1 419 799 A1 sowie der US 2009/080603 A1 beschrieben.

Die Bestrahlung eines endlichen Zielvolumens mittels Röntgenstrahlen aus verschiedenen Raumrichtungen, wie es beispielsweise zum Zwecke der Bildgebung bei diagnostischen Verfahren oder der Dosissynthese bei therapeutischen Verfahren durchgeführt wird, erfordert üblicherweise eine möglichst homogene Bruttoausleuchtung des Zielvolumens.

Da die üblicherweise eingesetzten Röntgenquellen eine bevorzugte Strahlrichtung besitzen, entlang derer sich das Dosismaximum befindet, und die Strahlrichtung üblicherweise zur Mitte des Zielvolumens hin ausgerichtet ist, ist die Ausleuchtung der Randbereiche des Zielvolumens üblicherweise reduziert gegenüber der Mitte des Zielvolumens.

Bisher wird das Problem, so wie zum Beispiel in der DE 100 60 887 A1 dargelegt, bei Strahlentherapiegeräten und bei Bildgebungsgeräten durch einen Abschwächer, auch Abflachungsfilter (engl.: "beam flattening filter") genannt, gelöst, der die zentralen Anteile der Strahlungskeule abschwächt und so das Profil des Röntgenstrahls homogenisiert.

Es ist die Aufgabe der Erfindung, ein medizinisches, mit Röntgenstrahlen arbeitendes Gerät bereitzustellen, das eine schnelle und effiziente Ausleuchtung eines Zielvolumens ermöglicht. Weiterhin ist es die Aufgabe der Erfindung, ein Verfahren bereitzustellen, mit dem es möglich ist, ein Zielvolumen schnell und effizient auszuleuchten.

Die Aufgabe der Erfindung wird gelöst durch die Merkmale der unabhängigen Ansprüche. Vorteilhafte Weiterbildungen der Erfindung finden sich in den Merkmalen der abhängigen Ansprüche.

Das erfindungsgemäße medizinische, mit Röntgenstrahlen arbeitende Gerät umfasst:
- eine Röntgenstrahlenquelle, mit der ein Röntgenstrahlkegel aussendbar ist, der ein inhomogenes Dosisprofil mit einem Intensitätsmaximum entlang eines Zentralstrahls aufweist,
- eine Rotationsvorrichtung, mit der die Röntgenstrahlenquelle um ein Isozentrum rotierbar ist,
wobei die Zentralachse des Röntgenstrahlenkegels paraxial am Isozentrum vorbei ausrichtbar ist, dadurch gekennzeichnet, dass das Gerät derart ausgebildet ist, dass im Betrieb bei einer Rotation der Rotationsvorrichtung um das Isozentrum die Zentralachse des Röntgenstrahlenkegels derart paraxial am Isozentrum vorbei gerichtet ist, dass die von unterschiedlichen Raumrichtungen ausgesendeten Zentralstrahlen tangential zu einem gedachten Kreis um das Isozentrum sind, so dass durch die Kombination aus inhomogenem Dosisprofil, paraxialer Ausrichtung sowie Rotation des Röntgenstrahlenkegels eine Homogenisierung des Dosisprofils in einem Zielvolumen und somit eine homogene Ausleuchtung erreicht ist.

Die Erfindung beruht auf der Erkenntnis, dass die Strahlungskeule einer Röntgenquelle, also der von der Röntgenquelle ausgesendete Röntgenstrahlenkegel, üblicherweise ein Maximum entlang des Zentralstrahls aufweist. Nun wird die Strahlungskeule der Röntgenquelle nicht mehr auf das Zentrum, sondern mehr auf den Randbereich gerichtet. Die Einstrahlung erfolgt also paraxial bzw. tangential aus verschiedenen Raumrichtungen.

Durch die Rotation der Quelle um das Zielvolumen wird dadurch insgesamt eine deutlich homogenere Ausleuchtung des gesamten

Volumens erreicht, auch wenn die Strahlungskeule selbst ein inhomogenes Dosisprofil zeigt. Die homogenere Ausleuchtung wird ermöglicht durch die Kombination aus inhomogenem Dosisprofil der Strahlungskeule, das sich um ein Dosismaximum entlang des Zentralstrahls abschwächt, aus exzentrischer Ausrichtung der Strahlungskeule sowie aus Rotation der Strahlungskeule.

Zur Homogenisierung der Ausleuchtung trägt also die geometrische Anordnung und Ausrichtung der Röntgenquelle zusammen mit der Rotation der Röntgenquelle bei.

Im Vergleich zu bisherigen Lösungen, bei denen die Homogenisierung weitgehend durch den Absorber bewerkstelligt wird, kann eine deutlich bessere Effizienz der Röntgenquelle erreicht werden.

Der Absorber kann nämlich so ausgestaltet werden, dass er weniger Strahlungsleistung absorbiert, da er nicht mehr allein für die Homogenisierung des Dosisprofils sorgen muss. Er kann sogar ganz weggelassen werden, sodass die volle Dosisleistung der Quelle auf das Zielvolumen trifft. In letzterem Fall ist im Röntgenstrahlengang frei von zur Homogenisierung dienenden Abflachungsfilter. Insgesamt kann so auf einen verlustbehafteten, strahlungsvernichteten Absorber verzichtet werden.

Dadurch erhöht sich die Dosisrate, mit der ein Zielvolumen ausgeleuchtet werden kann. Die Bestrahlungsdauer hängt von dem Bruch (gewünschte Dosis)/(lokale Dosisrate), über das Tumorvolumen gebildet, ab. Der zu bestrahlende Ort, an dem die minimale Dosisrate auftritt, also die am schwächsten beleuchtete Region, bestimmt daher die Bestrahlungsdauer. Daher kann mit dem erfindungsgemäßen medizinischen Gerät eine Zeitverkürzung erreicht werden.

Bei einer inhomogenen Dosisverteilung gilt zudem, dass gleichzeitig bestrahlte Orte mit höherer Dosisrate gegebenenfalls für einen Teil der Bestrahlungsdauer vor der Strahlung geschützt werden, da dort sonst dort eine zu hohe Dosis appliziert würde. Eine homogenere Dosisverteilung löst auch dieses Problem. Orte mit einer zu hohen Dosisrate müssen nicht nach kurzer Zeit ausgeblendet werden, z.B. mit einem Kollimator, damit man an anderem Ort die gewünschte Dosis bei geringerer Dosisrate applizieren kann.

Insbesondere kann das Gerät als Strahlentherapiegerät ausgebildet sein, beispielsweise mit einer therapeutischen Röntgenstrahlenquelle, die um ein Isozentrum rotiert werden kann. Ein Kollimator kann im Strahlengang des Röntgenstrahls vorhanden sein, mit dem dann das Strahlprofil des Röntgenstrahls seitlich begrenzt werden kann und so z.B. auf ein zu bestrahlendes Zielvolumen angepasst werden kann. So können bei einem zu bestrahlenden Objekt nur bestimmte Teilvolumina bestrahlt werden.

Das erfindungsgemäße Verfahren zum Betreiben eines medizinischen Diagnose-Geräts, aufweisend folgende Schritte:
- Bereitstellen einer Röntgenstrahlenquelle, mit der ein Röntgenstrahlenkegel ausgesendet wird, der entlang eines Zentralstrahls ein Intensitätsmaximum aufweist,
- Rotieren der Röntgenstrahlenquelle um ein Isozentrum, wobei die Zentralachse des Röntgenstrahlenkegels paraxial am Isozentrum vorbei gerichtet ist, derart, dass die von unterschiedlichen Raumrichtungen ausgesendeten Zentralstrahlen tangential zu einem gedachten Kreis um das Isozentrum sind, so dass durch die Kombination aus inhomogenem Dosisprofil, paraxialer Ausrichtung sowie Rotation des Röntgenstrahlenkegels eine Homogenisierung des Dosisprofils in einem Zielvolumen und somit eine homogene Ausleuchtung erreicht wird.

Bevorzugterweise ist im Strahlengang des Röntgenstrahls kein Abschwächungsfilter angeordnet. Der Strahlengang des Röntgenstrahls kann kollimiert werden, um das seitliche Profil zu formen.

Erfindungsgemäß wird der Röntgenstrahl, der ein Intensitätsmaximum im Zentralstrahl aufweist und der bei einem medizinischen, mit Röntgenstrahlen arbeitenden Gerät von einer Röntgenquelle von verschiedenen Raumrichtungen um ein Isozentrum ausgesendet wird, verwendet zur Dosishomogenisierung in einem Bereich um das Isozentrum, indem der Zentralstrahl des Röntgenstrahls bei jeder Raumrichtung seitlich an dem Isozentrum vorbei gerichtet wird.

Die vorangehende und die folgende Beschreibung der einzelnen Merkmale, deren Vorteile und deren Wirkungen bezieht sich sowohl auf die Vorrichtungskategorie als auch auf die Verfahrenskategorie, ohne dass dies im Einzelnen in jedem Fall explizit erwähnt ist; die dabei offenbarten Einzelmerkmale können auch in anderen als den gezeigten Kombinationen erfindungswesentlich sein.

Ausführungsformen der Erfindung mit Weiterbildungen gemäß den Merkmalen der abhängigen Ansprüche sind anhand der folgenden Zeichnung näher erläutert, ohne jedoch darauf beschränkt zu sein. Es zeigen:
- Fig. 1: das Prinzip der Dosiserzeugung bei einem Strahlentherapiegerät nach dem Stand der Technik,
- Fig. 2: das Prinzip der Dosiserzeugung bei einer Ausführungsform des erfindungsgemäßen Strahlentherapiegeräts.

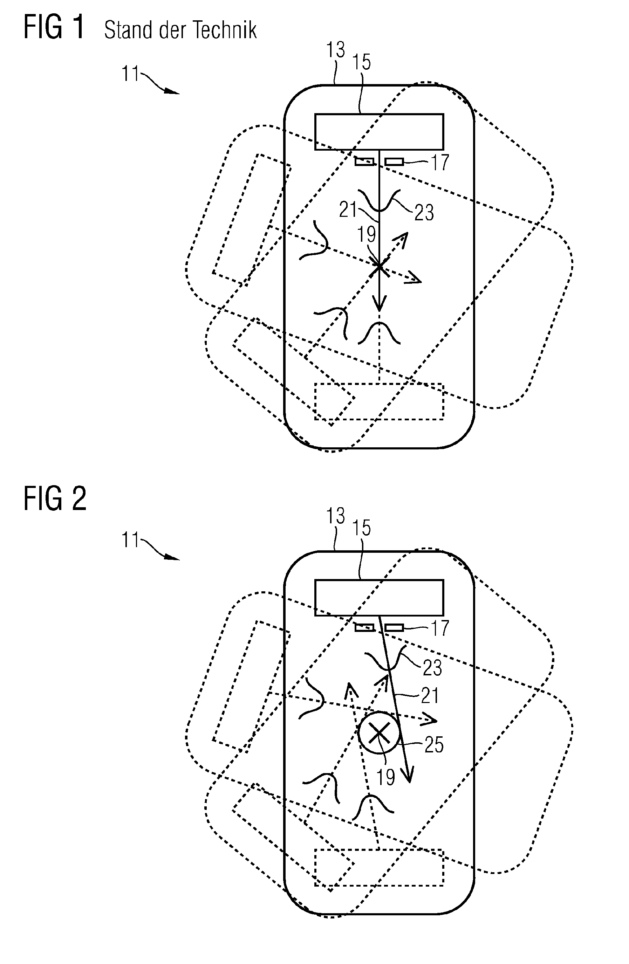

Anhand von Fig. 1 wird das Prinzip der Dosiserzeugung um ein Isozentrum bei einem Strahlentherapiegerät nach dem Stand der Technik erläutert.

Das Strahlentherapiegerät 11 umfasst eine rotierbare Gantry 13, mit der eine Röntgenstrahlenquelle 15 um das zu bestrahlende Objekt rotiert werden kann. Von der Röntgenstrahlenquelle aus wird ein kegelförmiger Röntgenstrahl auf das Isozentrum 19 gerichtet. Der Röntgenstrahl wird durch einen Kollimator 17 seitlich begrenzt. Die Bestrahlung erfolgt üblicherweise aus verschiedenen Richtungen unter Rotation der Röntgenquelle 15 um das Isozentrum 19, angedeutet durch die gestrichelt eingezeichneten Positionen der Gantry.

Der Zentralstrahl 21 des kegelförmigen Röntgenstrahls trifft dabei das Isozentrum 19. Der Röntgenstrahl weist dabei eine Dosisverteilung 23 - angedeutet durch die Gauss-artige Kurve - auf, die entlang des Zentralstrahls 21 ein Dosismaximum hat, welches zum Rand hin abnimmt. Wenn ein Zielvolumen mit dem Partikelstrahl bestrahlt wird, ist die Dosisrate im Isozentrum 19 daher am höchsten und schwächt sich zum Rand hin ab.

Deutlich erkennbar wird dieses Prinzip durch die applizierte Dosis, die durch die zwei entgegengesetzt gezeigten Einstrahlrichtungen erzeugt wird. Die Dosismaxima beleuchten das Zielvolumen an gleicher Stelle, so dass die Dosis, die durch die beiden Einstrahlrichtungen im Zielvolumen appliziert wird, die gleiche inhomogene Verteilung aufweist wie die inhomogene Dosisverteilung 23 im Röntgenstrahl.

Daher wird im Regelfall bei einem Strahlentherapiegerät 11 nach dem Stand der Technik ein Abflachungsfilter (hier nicht dargestellt) verwendet, der im Strahlengang des Röntgenstrahls angeordnet ist, und der das inhomogene Dosisprofil homogener macht. Dies geschieht jedoch auf Kosten von Strahlungsdosis, die im Abflachungsfilter vernichtet wird.

Fig. 2 zeigt das Prinzip der Dosiserzeugung bei einem erfindungsgemäßen Strahlentherapiegerät 11.

Der Zentralstrahl 21 der Röntgenquelle 15 ist nun nicht mehr auf das Isozentrum 19 ausgerichtet, sondern zielt paraxial am Isozentrum vorbei. Dies führt dazu, dass bei Rotation der Röntgenquelle 15 um das Isozentrum 19 die ausgesendeten Zentralstrahlen 21 tangential auf einem Kreis 25 um das Isozentrum liegen.

Die Dosis, die sich um das Isozentrum 19 bei einer derartigen geometrische Konfigurationen aufbaut, ist deutlich homogener verglichen mit einer Dosis, die bei Ausrichtung des Zentralstrahls 23 auf das Isozentrum 19 appliziert würde.

Deutlich erkennbar wird dies anhand der beiden entgegengesetzt eingezeichneten Einstrahlrichtungen. Das Dosismaximum, das durch die jeweiligen Einstrahlrichtungen appliziert wird, liegt nun nicht mehr im Isozentrum 19, sondern jeweils an entgegengesetzter Stelle des Isozentrums 19. Im Isozentrum 19 selbst summieren durch die entgegengesetzte Einstrahlung Dosisanteile auf, die deutlich unterhalb des Dosismaximums liegen. Durch die Addition der Dosisanteile wird im Isozentrum 19 eine Dosis appliziert, die deutlich näher am Dosismaximum liegt als die einzelnen Dosisanteile selbst. Insgesamt kann dadurch eine deutlich homogenere Dosis appliziert werden.

Im Idealfall kann daher sogar auf einen Abflachungsfilter gänzlich verzichtet werden. Es wird dann keine Dosisleistung im Abflachungsfilter vernichtet, wodurch sich das Zielvolumen mit einer höheren Dosisrate ausleuchten lässt, was eine Bestrahlungszeit deutlich verringert.

Zusätzlich kann auch hier ein Kollimator 17 vorgesehen sein, der die applizierte Dosis auf ein Zielvolumen angepasst und einschränkt.

Auch wenn das Prinzip anhand eines Strahlentherapiegerätes 11 erläutert wurde, kann das gleiche Prinzip, eine Dosishomogenisierung der um ein Isozentrum 19 aufgebauten Dosisverteilung zu erreichen, auf mit Röntgenstrahlen arbeitende Bildgebungsgeräte übertragen werden, wie z.B. ein Computertomograph (CT) oder ein Cone-Beam-CT.

### Bezugszeichenliste

- 11: Strahlentherapiegerät
- 13: Gantry
- 15: Röntgenstrahlenquelle
- 17: Kollimator
- 19: Isozentrum
- 21: Zentralstrahl
- 23: Dosisverteilung
- 25: Kreis

## Patentansprüche

1. Medizinisches, mit Röntgenstrahlen arbeitendes Gerät, aufweisend:
- eine Röntgenstrahlenquelle (15) , mit der ein Röntgenstrahlenkegel aussendbar ist, der ein inhomogenes Dosisprofil (23) mit einem Intensitätsmaximum entlang eines Zentralstrahls (21) aufweist,
- eine Rotationsvorrichtung (13) , mit der die Röntgenstrahlenquelle um ein Isozentrum (19) rotierbar ist,
wobei die Zentralachse des Röntgenstrahlenkegels paraxial am Isozentrum vorbei ausrichtbar ist, **dadurch gekennzeichnet, dass** das Gerät derart ausgebildet ist, dass im Betrieb bei einer Rotation der Rotationsvorrichtung um das Isozentrum die Zentralachse des Röntgenstrahlenkegels derart paraxial am Isozentrum vorbei gerichtet ist, dass die von unterschiedlichen Raumrichtungen ausgesendeten Zentralstrahlen tangential zu einem gedachten Kreis (25) um das Isozentrum sind, so dass durch die Kombination aus inhomogenem Dosisprofil, paraxialer Ausrichtung sowie Rotation des Röntgenstrahlenkegels eine Homogenisierung des Dosisprofils in einem Zielvolumen erreicht ist.

2. Medizinisches, mit Röntgenstrahlen arbeitendes Gerät, nach einem der Ansprüche 1, wobei das Gerät als Strahlentherapiegerät ausgebildet ist.

3. Medizinisches, mit Röntgenstrahlen arbeitendes Gerät, nach einem der Ansprüche 1 oder 2, wobei ein Kollimator im Strahlengang des Röntgenstrahlenkegels vorhanden ist zur seitlichen Begrenzung des Strahlprofils des Röntgenstrahls.

4. Medizinisches, mit Röntgenstrahlen arbeitendes Gerät, nach einem der Ansprüche 1 bis 3, wobei im Strahlengang des Röntgenstrahlenkegels kein zur räumlichen Dosishomogenisierung dienender Abschwächungsfilter angeordnet ist.

5. Verfahren zum Betreiben eines bildgebenden medizinischen Diagnose-Geräts zum Zwecke der Bildgebung, aufweisend folgende Schritte:
- Bereitstellen einer Röntgenstrahlenquelle, mit der ein Röntgenstrahlenkegel ausgesendet wird, der entlang eines Zentralstrahls ein Intensitätsmaximum aufweist,
- Rotieren der Röntgenstrahlenquelle um ein Isozentrum,
**dadurch gekennzeichnet, dass** die Zentralachse des Röntgenstrahlenkegels paraxial am Isozentrum vorbei gerichtet ist, derart, dass die von unterschiedlichen Raumrichtungen ausgesendeten Zentralstrahlen tangential zu einem gedachten Kreis um das Isozentrum sind, so dass durch die Kombination aus inhomogenem Dosisprofil, paraxialer Ausrichtung sowie Rotation des Röntgenstrahlenkegels eine Homogenisierung des Dosisprofils in einem Zielvolumen erreicht wird.

6. Verfahren nach Anspruch 5 , wobei im Strahlengang des Röntgenstrahlenkegels kein zur räumlichen Dosishomogenisierung dienender Abschwächungsfilter angeordnet ist.

7. Verfahren nach einem der Ansprüche 5 bis 6, wobei der Röntgenstrahlenkegel kollimiert wird.

## Claims

1. Medical device operating with x-rays, comprising:
- an x-ray radiation source (15), with which an x-ray radiation cone can be emitted, which has an inhomogenous dose profile (23) with a maximum intensity along a central beam (21),
- a rotation apparatus (13), with which the x-ray radiation source can be rotated about an isocenter (19),
wherein the central axis of the x-ray radiation cone can be aligned paraxially past the isocenter, **characterised in that** the device is embodied such that during operation, upon rotation of the rotation apparatus around the isocenter, the central axis of the x-ray radiation cone is aligned paraxially past the isocenter such that the central beams emitted from different spatial directions are tangential to an imaginary circle (25) around the isocenter so that a homogenisation of the dose profile is achieved in a target volume by combining inhomogeneous dose profile, paraxial alignment and rotation of the x-ray radiation cone.

2. Medical device operating with x-rays according to one of claims 1, wherein the device is embodied as a radiation therapy device.

3. Medical device operating with x-rays according to one of claims 1 or 2, wherein a collimator exists in the radiation path of the x-ray radiation cone for lateral delimitation of the beam profile of the x-ray beam.

4. Medical device operating with x-rays according to one of claims 1 to 3, wherein no beam flattening filter which is used for spatial dose homogenisation is arranged in the radiation path of the x-ray radiation cone.

5. Method for operating a medical imaging diagnostics device for the purpose of imaging, comprising the following steps:
- providing an x-ray radiation source, with which an x-ray radiation cone is emitted, which has a maximum intensity along a central beam,
- rotating the x-ray radiation source about an isocenter,
**characterised in that** the central axis of the x-ray radiation cone is directed paraxially past the isocenter such that the central beams emitted from different spatial directions are tangential to an imaginary circle around the isocenter, so that a homogenisation of the dose profile is achieved in a target volume by combining inhomogeneous dose profile, paraxial alignment and rotation of the x-ray radiation cone.

6. Method according to claim 5, wherein no beam flattening filter used for spatial dose homogenisation is arranged in the radiation path of the x-ray radiation cone.

7. Method according to one of claims 5 to 6, wherein the x-ray radiation cone is collimated.

## Revendications

1. Appareil médical à rayons X comportant :
- une source ( 15 ) de rayons x, par laquelle peut être émis un faisceau conique de rayons x, qui a un profil ( 23 ) de dose non homogène, ayant un maximum d'intensité le long d'un rayon ( 21 ) central,
- un dispositif ( 13 ) de mise en rotation, par lequel la source de rayons x peut tourner autour d'un isocentre ( 19 ), l'axe central du faisceau conique de rayons x pouvant être orienté paraxialement à l'isocentre, **caractérisé en ce que** l'appareil est constitué de manière à ce que, en fonctionnement pour une rotation du dispositif de mise en rotation autour de l'isocentre, l'axe central du faisceau conique de rayons x soit dirigé paraxialement à l'isocentre, de manière à ce que les rayons centraux émis de directions dans l'espace différentes soient tangents à un cercle ( 25 ) imaginaire autour de l'isocentre, de manière à ce que, par la combinaison d'un profil de dose non homogène, d'une orientation paraxiale, ainsi que d'une rotation du faisceau conique de rayons x, on obtienne une homogénéisation du profil de dose dans un volume cible.

2. Appareil médical à rayons x suivant l'une des revendications 1, dans lequel l'appareil est constitué en appareil de radiothérapie.

3. Appareil médical à rayons x suivant l'une des revendications 1 ou 2, dans lequel il y a un collimateur, dans le trajet des rayons du faisceau conique de rayons x, pour délimiter latéralement le profil du faisceau de rayons x.

4. Appareil médical à rayons x suivant l'une des revendications 1 à 3, dans lequel il y a, dans le trajet des rayons du faisceau conique de rayons x, un filtre d'atténuation servant à homogénéiser la dose dans l'espace.

5. Procédé pour faire fonctionner un appareil médical de diagnostic donnant une image en vue de donner une image, ayant les stades suivants :
- on se procure une source de rayons x, par laquelle on émet un faisceau conique de rayons x, qui a un maximum d'intensité le long d'un rayon central,
- on fait tourner la source de rayons x autour d'un isocentre, **caractérisé en ce que** l'axe central du faisceau conique de rayons x est dirigé paraxialement à l'isocentre, de manière à ce que les rayons centraux émis de directions différentes dans l'espace soient tangents à un cercle imaginaire autour de l'isocentre, de manière à obtenir, par la combinaison d'un profil de dose non homogène, d'une orientation paraxiale, ainsi que d'une rotation du faisceau conique de rayons x, un homogénéisation du profil de dose dans un volume cible.

6. Procédé suivant la revendication 5, dans lequel il y a, dans le trajet des rayons du faisceau conique de rayons x, un filtre d'atténuation servant à homogénéiser la dose dans l'espace.

7. Procédé suivant l'une des revendications 5 à 6, dans lequel le faisceau conique de rayons x est collimaté.
